## Europäisches Patentamt

## European Patent Office

## Office européen des brevets

(11) Publication number: **0 352 839**
**A2**

# (12) EUROPEAN PATENT APPLICATION

(21) Application number: **89201822.7**

(22) Date of filing: **07.07.89**

(51) Int. Cl.⁴: **C12N 15/16 , C12P 21/02**

(30) Priority: **15.07.88 JP 177685/88**

(43) Date of publication of application:
**31.01.90 Bulletin 90/05**

(84) Designated Contracting States:
**DE ES IT NL SE**

(71) Applicant: **NIPPON SHINYAKU COMPANY, LIMITED**
**14, Kisshoin Nishinosho Monguchicho**
**Minami-ku Kyoto-shi Kyoto 601(JP)**

(72) Inventor: **Yano, Junichi**
**840-86 Sahodai-2-chome**
**Nara 630(JP)**
Inventor: **Murai, Masatoshi**
**5-1-1643 Takahamacho**
**Ashiya 659(JP)**

(74) Representative: **Kupecz, Arpad et al**
**Octrooibureau Los en Stigter B.V. Postbox 20052**
**NL-1000 HB Amsterdam(NL)**

(54) **Process for producing hEGF.**

(57) In a genetic engineering method, a large amount of hEGF was secreted in a medium of high density E. coli culture utilizing the controlled fed-batch system. The constructed plasmid in the recombinant host encoded a series of DNA sequences for a trip promotor, an E. coli alkaline phosphatase signal peptide and a synthetic human EGF. The invention provides hEGF in a naturally occurring form which is applicable to an antiulcer drug.

```
                                    ──────── trp Promotor ────────
HindIII
AGCTTTGGCAAATATTCTGAAATGAGCTGTTGACAATTAATCATCGAACTAGTTAACTAG
.......AACCGTTTATAAGACTTTACTCGACAACTGTTAATTAGTAGCTTGATCAATTGATC

                    ┌──────────────────┐┌── E. coli alkaline phosphat-
                    │   SD  Sequence   ││
TACGCAAGTTCACGTAAAAAGGTATCGACAATGAAACAAAGCACTATTGCACTGGCACT
ATGCGTTCAAGTGCATTTTTCCATAGCTGTTACTTTGTTTCGTGATAACGTGACCGTGA

ase signal peptide gene
                                                  ┌── EcoRI
CTTACCGTTACTGTTTACCCCTGTGACAAAAGCG
GAATGGCAATGACAAATGGGGACACTGTTTTCGCTTAA
```

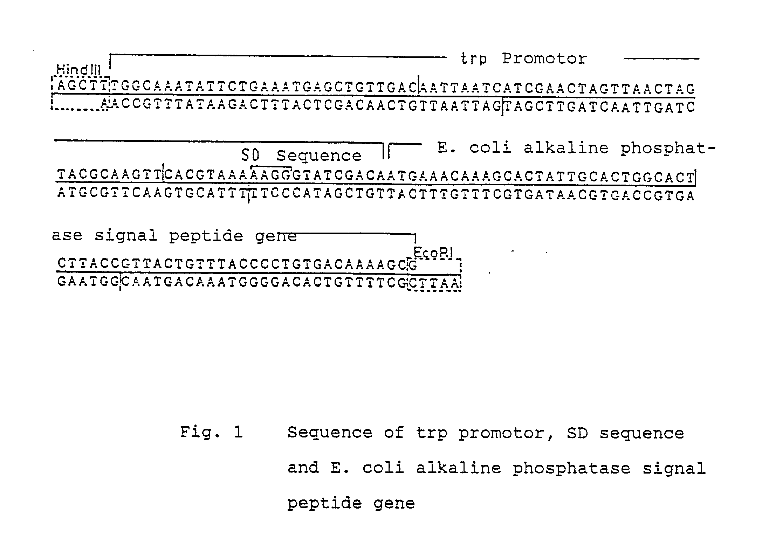

Fig. 1    Sequence of trp promotor, SD sequence and E. coli alkaline phosphatase signal peptide gene

# PROCESS FOR PRODUCING hEGF

(Field of Industrial Applicability)

The present invention relates to a process for producing human epidermal growth factor (hEGF) efficiently utilizing genetic engineering technique.

It has now been clarified that hEGF is the same substance as $\beta$-urogastrone. The present invention provides hEGF which is useful as drugs such as an anti-ulcer agent, etc., in a large scale in a simple manner.

[Prior Art]

Genetic engineering technique for performing the series of procedures of (1) through (4): (1) integrating a gene coding for an objective substance in a vector, (2) transforming host E. coli using this vector, (3) culturing the thus obtained transformant, and (4) recovering and collecting the objective substance from the culture solution, is already known. Various devices have been made to acquire trace proteins useful for human according to this technique. Details are described in:

Tacon, W., Carey, N., Emetage, S.: Molec. Gen. Genet., 177, 427 (1980)

Tacon, W., et. al. : Gene 23, 255 (1983)

Kawai, S., et. al. : J Ferment. Technol., 64, 503 (1986)

Oka, T., et. al. Proc. Natl. Acad. Sci. USA. 82, 7212 (1985) and others.

According to the latest technique, there is disclosed a method which comprises creating recombinant DNA bearing both a gene encoding an E. coli alkaline phosphatase gene-derived promoter and a gene encoding a signal peptide under its control and having also integrated a gene encoding hEGF thereby to for finally obtain hEGF (Japanese Published Unexamined Patent Application Nos. 003799/89 and 003800/89). In this method, for purposes of increasing the yield, various inventions have made during the incubation, for example; by culturing in a low temperature zone prior to inducing protein synthesis capability and in a high temperature zone after the induction of protein synthesis capability; or by culturing under weakly acidic or neutral conditions prior to inducing protein synthesis capability but under weakly alkaline conditions after the induction of protein synthesis capability. These inventions achieve good results.

[Problems to be solved by the Invention]

According to the prior art described above, the objective hEGF can be indeed produced in a yield to some extent. However, in the case of intending high density culture accompanied by secretion and expression in a medium, the prior art involves many defects. For example, the defects are that (1) the prior art cannot freely control the time when the transformed E. coli starts producing hEGF; (2) for this reason, it is impossible to sufficiently proliferate the transformed E. coli prior to initiating the production of hEGF; and (3) therefore, it is impossible for the transformed E. coli to control the progress of producing hEGF, in so called high density culture (which refers to culture by E. coli or more than 40 $OD_{550}$ in the specification).

In view of its principle, it was also impossible to apply the fed-batch system (technique for continuous culture while supplementing a medium during the culture) mandatorily required for high density culture.

The experiments performed by the present inventors reveal a tendency that proliferation of the transformed E. coli itself is inhibited, when transformed E. coli begins to produce hEGF. While the reason is not necessarily clear, it is assumed that a large stress would be borne on the transformed E. coli, taking into the phenomenon specific to hEGF that the produced hEGF does not remain in cytoplasm of the transformed E. coli or in periplasm (between the inner membrane and the outer membrane) but is secreted outside the cells. Based on the phenomenon, it is assumed to be because proliferation of the transformed E. coli itself would be inhibited at the same time when hEGF is produced.

From the foregoing, in order to realize high density culture, it was necessary to initiate the production of hEGF at that time when the transformed E. coli is sufficiently proliferated and for this reason, it was necessary to freely control the time of producing hEGF.

[Means for solving the Problems]

The gist of the present invention lies in the following points.

(1) A gene encoding a promoter for tryptophan (trp) operon is used as a promoter.

(2) By adding 3-indoleacrylic acid (IAA), the time period at which the transformed E. coli produces hEGF is controlled.

(3) In the culture, the fed-batch system in which a rate of supplementing a medium to be added is variable is efficiently used.

(4) Carbon sources of the supplementing medium are glucose or glycerol or glucose and glycerol in combination.

(5) Further in the fed-batch system (4) above, glucose is used and then glycerol is used, as carbon sources for the supplementing medium in the fed-batch system.

Hereafter the above points are described in detail.

When using as a promoter an E. coli alkaline phosphatase gene-derived promoter, hEGF productivity stops due to the presence of phosphate in the medium. Transformed E. coli proliferates and during this course, the phosphate is consumed and reduced to less than a definite concentration. Then, the E. coli alkaline phosphatase gene-derived promoter begins to act to initiate hEGF production. It was the prior art technique that the phenomenon occurred during the procedure is controlled by controlling pH and temperature of the culture solution thereby to enhance the hEGF productivity.

In the present invention, the aforesaid promoter of trp operon is used, instead of the E. coli alkaline phosphatase gene-derived promoter described above.

In the present invention, about 50 mg/l of tryptophan is previously added to medium. As transformed E. coli proliferates, tryptophan is consumed; when tryptophan is reduced to less than a definite concentration, the promoter of trp operon begins to act to initiate hEGF production. However, when tryptophan is sufficiently present in the medium, there is no chance that the promoter of the present invention would initiate hEGF production. Furthermore, as will be later shown in the examples, particularly the transformed E. coli has properties that when it secrets hEGF, its proliferation rate is weakened or its proliferation is discontinued.

From these facts, it has been found that the secretion expression plasmid vector bearing the trp promoter used in the present invention is extremely suited to utilize for high density culture of transformed E. coli later described since its expression can be strictly controlled easily. That is, in order to increase the secretion amount of secreted hEGF to more than 150 mg/l, incubation is performed in the presence of tryptophan; when transformed E. coli is proliferated in a sufficiently high density, IAA is immediately added and the product can be recovered from the medium in a short time (10 to 18 hours).

Further by addition of glycerol into medium in the fed-batch system in place of glucose or by changing glucose to glycerol on the way of addition, it is possible to ingeniously control the timing for initiating the action of trp promoter, as will be later described.

IAA is a substance having a structure similar to that of tryptophan and has affinity to repressor protein stronger than that of tryptophan. Accordingly, IAA predominantly binds to repressor to prevent repressor from being bound to promoter (operator). Therefore, by incorporating IAA into medium, this promoter begins to act even in such a state that tryptophan is present in medium.

From the foregoing, it is understood that hEGF production can be initiated by addition of IAA in the present invention at any stage of the culture. The important feature of the present invention actually resides in this point.

It is known that when proliferation of transformed E. coli is enhanced, the subsequent hEGF production is proportionally enhanced. The density of transformed E. coli in the medium can be increased by designing the medium itself or by applying the fed-batch system. Therefore, only by starting hEGF production after proliferation of transformed E. coli is enhanced, hEGF can be collected so efficiently.

In the present invention, techniques conventionally used in ordinary genetic engineering can be appropriately used. For example, as host E. coli, K12-derived E. coli strain widely used is usable. Further as the vector, commercially available plasmid can be recombined by genetic engineering technique to provide for use. More specifically, the following E. coli strains can be used in the present invention.

JM101, TB1, HB101, RR1, DH1, MM294, C600galK⁻, Y1090 (pMC9-deficient strain).

In addition, derivative strains thereof can also be used.

In the present invention, for example, the following plasmid and phage DNA can be used.

pDR540 (manufactured by Pharmacia Fine Chemicals Inc.), m13mp18 and mp19 (manufactured by Takara Shuzo Co., Ltd.).

Further in the present invention, for example, the following media can be used as basal medium and supplementing medium.

LB medium (10 g/l bacto-trypton, 5 g/l yeast extract, 5 g/l sodium chloride)

3

M9 medium (6 g/l disodium hydrogenphosphate, 3 g/l potassium dihydrogenphosphate, 0.5 g/l sodium chloride ammonium chloride, 2 mM magnesium sulfate, 0.1 mM calcium chloride)

4YTM9 medium (M9 medium supplemented with the following components: 32 g/l bacto-trypton, 20 g/l yeast extract)

The medium can be prepared by adding agar to plate medium in a concentration of 1.5% and to M13 phage upper layer medium in a concentration of 0.6%. For culturing E. coli containing all plasmids, ampicillin is added to the medium in 100 mg/l thereby to prevent the plasmids from being cured.

As restriction enzymes and DNA modifying enzymes used in the present invention, those used in ordinary genetic engineering can be appropriately used. For example, enzymes manufactured by Takara Shuzo Co., Ltd., Toyobo Co., Ltd., Pharmacia Fine Chemicals Inc. and Boehringer Mannheim. In the present invention, these enzymes can be used in accordance with manuals and publications (Molecular Cloning; a laboratory manual. Cold Spring Harbor Laboratory, Cold Spring Harbor, New York).

Further for transformation of E. coli, purification of plasmid and purification of phage DNA in the present invention, techniques used in ordinary genetic engineering can be appropriately used. Alternatively, they may follow manuals and publications (Molecular Cloning).

The oligonucleotide in the present invention can be synthesized by appropriately using techniques conventionally applied to ordinary DNA synthesis (for example, triester method). On the other hand, the oligonucleotide can also be synthesized using commercially available DNA synthesizer (Model 380B, manufactured by Applied Biosystem Inc.).

Further for ligation of the oligonucleotide in the present invention, techniques used in ordinary genetic engineering can be appropriately used. Specifically, for example, 1 $\mu$g of each of the synthetic oligonucleotide is phosphorylated in 20 $\mu$l of linker kinase buffer (by Molecular Cloning) and then mixed. After heating at 70°C for 10 minutes, the system is gradually cooled to room temperature over about an hour. Next, the same volume of 1-fold concentration linker kinase buffer as the mixture is added to the mixture and T4 DNA ligase is then added thereto. The mixture is allowed to stand overnight at 16°C. Next, the mixture is heated at 70°C for 10 minutes to inactivate T4 DNA ligase. Thereafter, the buffer is exchanged by the ethanol precipitation method. After cleaving with restriction enzymes at both termini so as to form the objective fragment, fractionation is carried out by 8% polyacrylamide slab gel electrophoresis. From the gel containing the objective DNA fragment, DNA is purified by extraction with buffer (by Molecular Cloning). Such a technique is a typical example of ligation of oligonucleotide in the present invention.

To determine the amount of hEGF which is the objective product in the present invention, techniques used in ordinary genetic engineering can be appropriately used. Specifically, the hEGF amount can be determined by radioimmunoassay (using the kit manufactured by Amersham Inc.) using, for example, commercially available hEGF (manufactured by Wakunaga Pharmaceutical Co., Ltd., or Amersham Inc.) as standard.

[Examples]

Hereafter the present invention is described in more detail, by referring to reference examples and examples.

Reference Example 1

[Production of secretion expression plasmid]

(1) Synthesis of trp promoter, SD sequence and E. coli alkaline phosphatase signal peptide gene:

The trp promoter and SD sequence (Shine-Dargarno ribosome-bound site) utilized were set from T at the -60 position to A at the +26 position when A as the transcription initiation site was made +1. At the 5'-end of T at the -60 position, Hind III cleavage site was provided, taking ligation with vector into account. The E. coli alkaline phosphatase signal peptide gene followed naturally occurring sequence but taking ligation with vector and ligation with hEGF gene into account, GTG of initiation codon was modified to ATG, GCT at the C-terminal was modified to GCG and EcoR I cleavage site was provided at the 3'-end. The foregoing sequence was synthesized by dividing into 8 oligonucleotides, which were ligated in a conventional manner.

4

The product was purified as Hind III-EcoR I DNA fragment. Further this fragment was inserted between Hind III-EcoR I cleavage sites of M13mp18 phage replicative form DNA and its nucleotide sequence was confirmed by the dideoxy sequencing method (Fig. 1).

## (2) Synthesis of hEGF gene

The sequence of hEGF gene was devised based on the amino acid sequence of natural hEGF. Upon device of the sequence, the following 3 points were taken into account: use of codon used with high frequency in E. coli, removal of repeating sequence that can take a secondary structure and provision of Sph I cleavage site in the middle of gene necessary for gene ligation.

Specifically, hEGF gene was produced as the EcoR I-Sac I DNA fragment composed of 21 oligonucleotides.

Firstly, 11 oligonucleotides constituting the former part of the EcoR I-Sac I DNA fragment was synthesized and ligated in a conventional manner, which was then inserted between the EcoR I-Sph I cleavage site of M13mp18 phage replicative form DNA. The nucleotide sequence was confirmed by the dideoxy sequencing method.

Next, 10 oligonucleotides constituting the latter part of the Sph I-Sac I DNA fragment was synthesized and ligated in a conventional manner, which was then inserted between the Sph I-Sph I cleavage site of M13mp18 phage replicative form DNA. The nucleotide sequence was confirmed by the dideoxy sequencing method.

From the respective recombinant M13 phage replicative form DNAs, the EcoR I-Sph I DNA fragment and the Sph I-Sac I DNA fragment were excised and ligated with each other to produce hEGF gene.

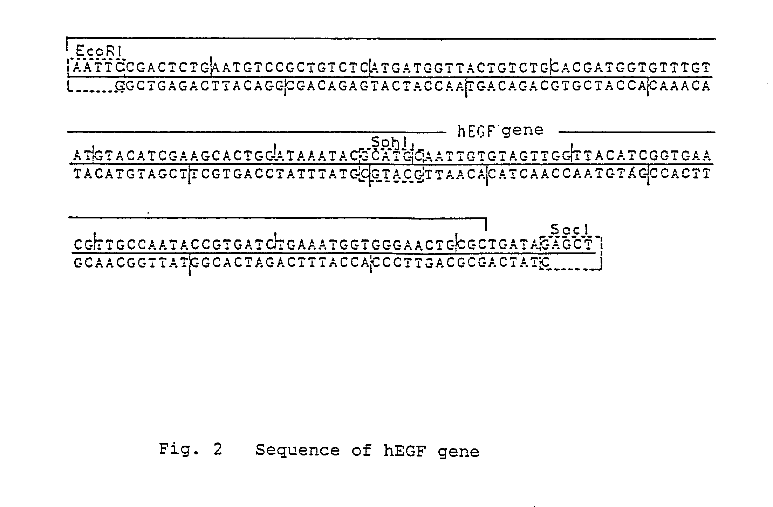

In the completed hEGF gene, the 5′-end constitutes the EcoR I cleavage site and this portion corresponds to asparagine of the N-terminal amino acid. Its 3′-end constitutes the Sac I cleavage site and is in the form that termination codons TGA and TAG follows just therebefore. The EcoR I-Sac I DNA fragment of the ligated hEGF gene was inserted into the EcoR I-Sac I cleavage site of M13mp18 phage replicative form DNA. The nucleotide sequence was again confirmed by the dideoxy sequencing method(Fig. 2).

## (3) Production of secretion expression plasmid

Firstly, the EcoR I cleavage site of pDR540 (manufactured by Pharmacia Fine Chemicals Inc.) was erased in a conventional manner using DNA polymerase Klenow fragment and T4 ligase to produce pDR540E⁻ plasmid.

Next, linker synthesized and ligated in a conventional manner was inserted into the BamH I cleavage site of this pDR540E⁻ DNA and, cleavage sites with EcoR I, Xho I, Sac I and Pvu II were newly introduced therein.

Furthermore, trp A terminator sequence (manufactured by Pharmacia Fine Chemicals Inc.) was inserted into this Pvu II cleavage site to prepare pATGt plasmid. The EcoR I-Sac I DNA fragment containing synthetic hEGF gene was inserted between the EcoR I cleavage site and the Sac I cleavage site of this pATGt plasmid DNA to prepare pBT19 plasmid.

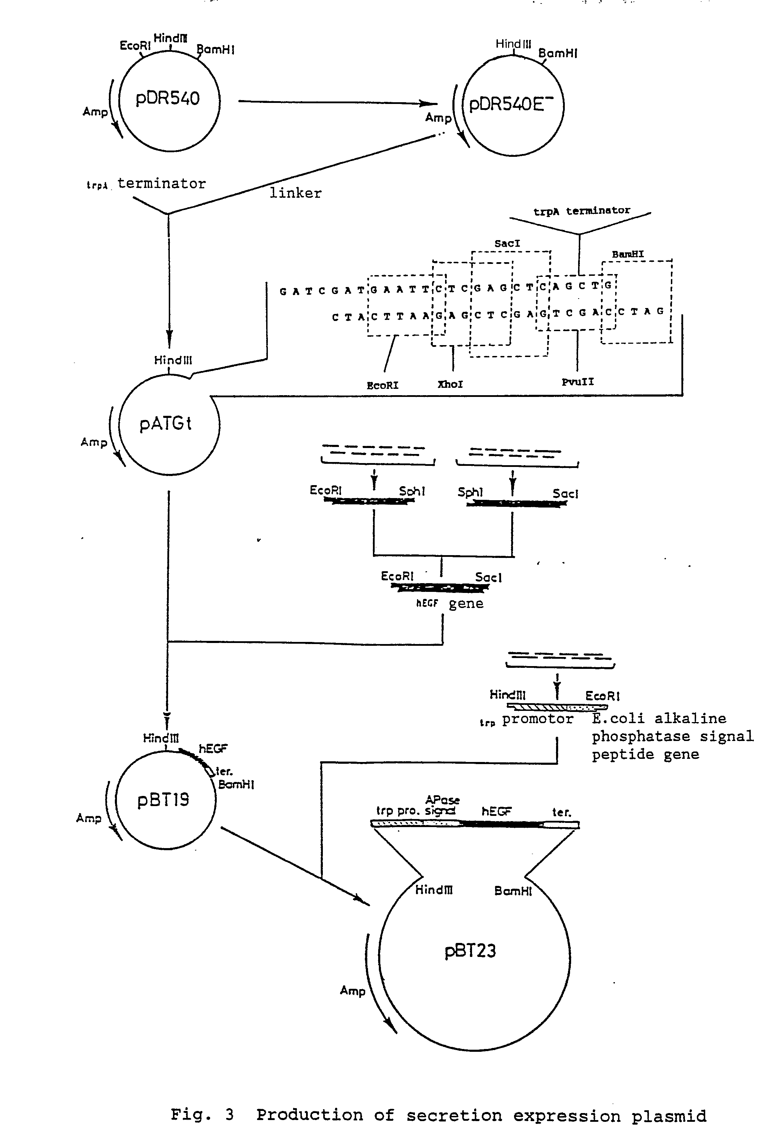

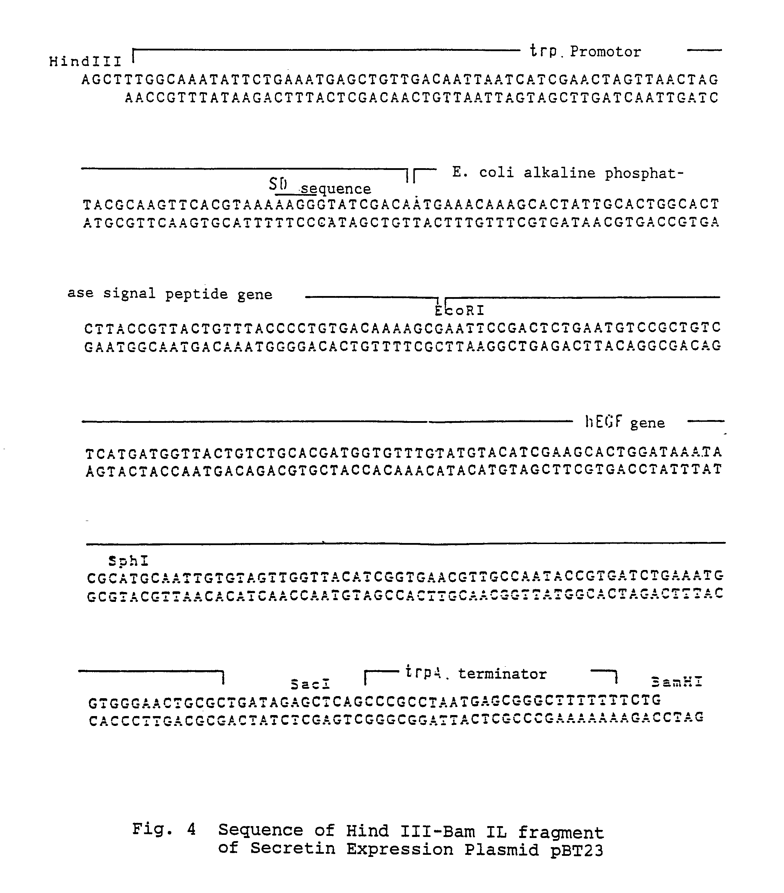

Next, the Hind III-EcoR I DNA fragment containing trp promoter, SD sequence and E. coli alkaline phosphatase signal peptide gene was inserted between the Hind III cleavage site and the EcoR I cleavage site of pBT19 to produce hEGF secretion expression plasmid pBT23 (Fig. 3 and Fig. 4).

Reference Example 2

[Expression of hEGF and its distribution]

Using pBT23 , E. coli C600galK⁻ was transformed to obtain transformed E. coli containing pBT23.

This pBT23-containing C600galK⁻ strain was shake cultured in 0.1 liter of LB medium overnight, which was made preculture solution. The preculture solution, 4 ml, was inoculated (2% inoculation) on 0.2 liter of M9 medium (containing 5 g/l Casamino acid, 2 g/l glucose, 10 mg/l vitamin $B_1$ and 100 mg/l ampicillin) charged in a shake flask of a 0.5 liter volume followed by shake culture at 37°C at 125 times/minute. At the exponential growth plase (in this case, $OD_{550}$ was about 0.1), 3-indoleacrylic acid (IAA) was added in a concentration of 20 mg/l to induce trp promoter. The sample was taken 1, 2, 4, 6, 8 and 24 hours after the initiation of culture and centrifuged to separate the medium and the cell component from each other. From the cells, their periplasm fraction was further separated by the osmotic shock procedure. The amount of hEGF in the medium and the periplasm was determined by radioimmunoassay (kit manufactured by Amersham Inc.). The results are shown in Table 1.

Table 1

| Distribution of hEGF | | |
|---|---|---|
| Culture Time | Amount of hEGF Periplasm | (mg/l) Medium |
| 1 | 0.011 | 0.017 |
| 2 | 0.016 | 0.030 |
| 4 | 0.022 | 0.190 |
| 6 | 0.017 | 0.430 |
| 8 | 0.014 | 0.490 |
| 24 | 0.004 | 0.730 |

The hEGF amount in the medium reached the maximum or 0.73 mg/l, 24 hours after the initiation of the culture. The hEGF accumulated in the periplasm reached the maximum 4 hours after the culture was initiated but its amount was about 0.02 mg/l at maximum. hEGF was reduced and almost lost 24 hours after.

From these facts, it was confirmed that the secreted hEGF was mostly present in the medium. Thus, in the following runs, the amount of hEGF produced was measured only in the medium.

Reference Example 3

[Study on host E. coli strain]

Using pBT23 DNA, E. coli HB101, RR1, DH1, MM294, Y1090 (pMC9-deficient strain), JM101 and TB1 strains were transformed to obtain E. coli transformants containing the respective pBT23.

The transformants were cultured under the same conditions as in Reference Example 2, also including the previous C600galK⁻ transformant. Induction of trp promoter with IAA was carried out 9.5 hours after the culture was initiated. At this point in time and 24 hours after, the amount of hEGF in the medium was measured (Table 2).

Table 2

| Amount of hEGF in various E. coli transformants | | |
|---|---|---|
| | Amount of hEGF (mg/l) | |
| E. coli Strain | 9.5 Hours After | 24 Hours After |
| C600galK⁻ | 0.54 | 1.25 |
| HB101 | 0.54 | 1.25 |
| RR1 | 1.23 | 0.93 |
| DH1 | 0.64 | 0.45 |
| MM294 | 0.28 | 0.50 |
| Y1090 (pMC9-deficient strain) | 0.03 | 0.02 |
| JM101 | 0.81 | 0.67 |
| TB1 | 2.30 | 4.00 |

Among the 8 E. coli transformants, TB1 provides the largest production amount and reached 4 mg/l. Then, C600galK⁻ and HB101 were the second largest and both reached 1.25 mg/l. These strains were considered to be suitable for producing of hEGF.

Reference Example 4

[Study on timing for inducing trp promoter]

Using pBT23-containing HB101 transformant, culture was carried out under the same conditions as in Reference Example 2. IAA was added in a concentration of 20 mg/l in the intermediate, later and stationary stages of exponential growth phase, respectively to examine the hEGF amount in the medium. The results are shown in Table 3.

Table 3

| Timing for inducing trp promoter and hEGF production Amount of hEGF (mg/l) | | | |
|---|---|---|---|
| Culture Time | Exponential Growth Plase | | |
| | Intermediate Stage | Later Stage | Stationary phase |
| 0.5 | < 0.08 | < 0.08 | < 0.08 |
| 1.5 | < 0.08 | < 0.08 | < 0.08 |
| 2.5 | < 0.08 (added with IAA) | < 0.08 | < 0.08 |
| 3.5 | 0.18 | 0.16 | 0.14 |
| 4.5 | 0.65 | 0.43 (added with IAA) | 0.33 |
| 5.5 | 1.69 | 0.83 | 0.63 |
| 6.5 | 2.28 | 0.98 | 0.90 |
| 7.5 | 2.63 | 1.10 | 1.10 |
| 9.5 | 2.41 | 1.63 | 1.66 |
| 24.0 | 5.75 | 8.95 | 8.95 |

It was noted that the addition of IAA in the later stage of the exponential growth phase or stationary phase produced larger amounts of hEGF production.

Reference Example 5

Using pBT23-containing HB101 transformant, culture was carried out under the same conditions as in Reference Example 2 (except that media shown in Table 4 were used). When 4YTM9, M9-1 and M9-2 were used as media, IAA was added 5.5 hours after and when M9-3 and M9-4 were used, 9 hours after, in a concentration of 20 mg/l. The hEGF amount in each medium was measured 9 and 24 hours after the culture was initiated.

Table 4

| Composition of medium | | | | |
|---|---|---|---|---|
| 4YM9 | M9-1 | M9-2 | M9-3 | M9-4 |
| M9 medium<br>Bacto-trypton 32 g/l<br>Yeast extract 20 g/l | ←<br>Casamino acid 10 g/l<br>Glucose 5 g/l<br>Vitamin B₁ 10 mg/l | ←<br>5 g/l<br>←<br>← | ←<br>←<br>2 g/l<br>← | ←<br>2 g/l<br>←<br>← |
| Ampicillin 100 mg/l | ← | ← | ← | ← |

Table 5

| Composition of medium and<br>amount of hEGF production | | |
|---|---|---|
| | Amount of hEGF<br>(mg/l) | |
| Medium | 9 Hours<br>After | 24 Hours<br>After |
| 4YTM9 | 0.04 | 0.09 |
| M9-1 | 0.68 | 0.70 |
| M9-2 | 1.98 | 1.69 |
| M9-3 | 3.03 | 7.25 |
| M9-4 | 0.91 | 2.38 |

When M9-3 medium (containing 5 g/l Casamino acid and 2 g/l glucose) was used, the hEGF amount reached the maximum (7.25 mg/l). It was also noted that in the medium composed mainly of natural components (for example, yeast extract, Bacto-trypton) containing large amounts of tryptophan, the hEGF production amount was small.

Reference Example 6

[Control of trp promoter by tryptophan in medium]

The culture in Reference Examples described above contains no tryptophan in the media and is the natural induction induction system in which induction of trp promoter occurs at the time when tryptophan derived from the preculture solution was reduced to less than a definite concentration as E. coli was proliferated. In this system, addition of the inducer IAA could increase the production of hEGF but could not switch the trp promoter induction on directly.

Thus, tryptophan was previously added to medium to see if induction from trp promoter could be inhibited and assuming this is the case, to see if it was possible to release the inhibition by adding IAA.

The amounts of tryptophan added were 1, 3, 10, 20, 30 and 40 mg/l and IAA was also added in concentrations shown in Table 6.

Using pBT-23 containing HB101 transformant, culture was carried out under the same conditions as in Reference Example 2. IAA was added 5 hours after the culture was initiated. The hEGF amount in the medium was determined. The results are shown in Table 6.

Table 6

| | No. | Concentration of Tryptophan (mg/l) | Concentration of IAA (mg/l) | Amount of hEGF (mg/l) | | | | | | |
|---|---|---|---|---|---|---|---|---|---|---|
| Concentration of tryptophan in medium and hEGF production amount | | | | | | | | | | |
| | | | | 1 Hour After | 3 Hours After | 5 Hours After | 7 Hours After | 9 Hours After | 11 Hours After | 24 Hours After |
| | ( 1) | 0 | 0 | <0.08 | 0.22 | 1.44 | 2.15 | 2.68 | 3.75 | 6.35 |
| | ( 2) | 0 | 20 | <0.08 | 0.27 | 0.97 | 1.46 | 1.75 | 2.40 | 9.75 |
| | ( 3) | 1 | 0 | <0.08 | 0.27 | 1.18 | 1.84 | 2.45 | 4.33 | 7.75 |
| | ( 4) | 1 | 20 | <0.08 | 0.26 | 1.16 | 2.04 | 2.31 | 3.80 | 12.50 |
| | ( 5) | 3 | 0 | <0.08 | 0.29 | 1.09 | 1.74 | 3.45 | 4.58 | 9.50 |
| | ( 6) | 3 | 20 | <0.08 | 0.27 | 1.09 | 1.94 | 2.25 | 3.25 | 10.50 |
| | ( 7) | 10 | 0 | <0.08 | <0.08 | 0.66 | 0.96 | 1.71 | 2.68 | 8.75 |
| | ( 8) | 10 | 20 | <0.08 | 0.08 | 0.69 | 1.13 | 1.65 | 2.55 | 8.00 |
| | ( 9) | 20 | 0 | <0.08 | <0.08 | 0.35 | 0.46 | 0.53 | 0.67 | 1.03 |
| | (10) | 20 | 20 | <0.08 | <0.08 | 0.35 | 0.71 | 1.31 | 2.00 | 7.90 |
| | (11) | 20 | 40 | <0.08 | <0.08 | 0.45 | 0.82 | 1.38 | 2.01 | 7.30 |
| | (12) | 20 | 80 | <0.08 | <0.08 | 0.45 | 0.57 | 1.04 | 1.34 | 3.60 |
| | (13) | 30 | 0 | <0.08 | <0.08 | 0.42 | 0.47 | 0.60 | 0.86 | 1.33 |
| | (14) | 30 | 80 | <0.08 | <0.08 | 0.42 | 0.79 | 1.13 | 1.30 | 2.09 |
| | (15) | 40 | 0 | <0.08 | <0.08 | 0.43 | 0.51 | 0.60 | 0.81 | 1.41 |
| | (16) | 40 | 80 | <0.08 | <0.08 | 0.37 | 0.60 | 1.07 | 1.38 | 2.15 |

When the concentration of tryptophan added in the medium was less than 10 mg/l, the hEGF production amount was 6 to 10 mg/l, whereas when the concentration of tryptophan was 20 to 40 mg/l, the hEGF production amount was as greatly reduced as 1 to 1.5 mg/l. It was thus noted that tryptophan of more than the definite concentration inhibited production of hEGF.

Further in the case that the concentration of tryptophan was 20 mg/l, the hEGF production amount increased by about 8 times by adding 20 or 40 mg/l of IAA. When 80 mg/l of IAA was added, however, the hEGF production amount increased merely by about 4 times. When the concentration of tryptophan was 30 to 40 mg/l, an increase of the hEGF production amount by addition of 80 mg/l of IAA was as small as about 1.5 time.

From the foregoing results, it was confirmed that the addition of tryptophan in an appropriate amount (20 mg/l in this example) inhibited the induction from trp promoter and the addition of IAA (20 to 40 mg/l) released the inhibition by tryptophan to switch trp promoter on. It was also noted that the addition of tryptophan in a trace amount promoted proliferation of E. coli transformants (the cell amount becomes 1.5 to 2 times by adding 10 to 40 mg/l of tryptophan). It was further noted that the addition of IAA in an excess amount such as more than 40 mg/l inhibited proliferation of E. coli, resulting in reduction in the hEGF production amount.

The foregoing results are summarized below.

(1) Almost all of the objective hEGF are secreted in the culture solution by sufficient incubation.

(2) HB101, TB1 or C600galK⁻ are suitable as host E. coli strains.

(3) Induction of trp promoter by IAA is optimum when added at the later growth phase.

(4) Medium supplemented with 5 g/l Casamino acid, 2 g/l glucose and 10 mg/l vitamin $B_1$ is appropriate as the medium.

(5) By adding IAA, induction of trp promoter is possible, even though tryptophan is present in the medium.

Assuming that high density culture in a jar fermenter is enabled in a large scale using these facts as indices, the following experiments were carried out. Further in order to withstand the large scale experiment, purification of hEGF in the culture supernatant of E. coli was examined and the following method was discovered.

Experiment 1

[Purification of hEGF]

(1) Adsorption of cation exchange resin (BIO-REX7O: manufactured by Biorad Inc.) (pH 3.0) → elution (ammonium acetate, pH 8.0) → dialysis (ammonium acetate, pH 5.5)

(2) Anion exchange column chromatography (Q-Sepharose Fast Flow: manufactured by Pharmacia Fine Chemicals Inc., (ammonium acetate, pH 5.5)

(3) Reversed phase ($C_{18}$) column chromatography (PepRPC: manufactured by Pharmacia Fine Chemicals Inc., 0.1% trifluoroacetic acid/acetonitrile) - freeze drying

According to this method, the overall steps can be performed in 2 to 4 days. In addition, the method does not involve gel filtration chromatography conventionally used so that scaling up is easy.

For example, 10 mg of purified hEGF could be obtained from 10 liters of the culture supernatant by this method.

The purified hEGF had the same amino acid sequence as in the natural one and had the same N-terminal amino acid sequence as in the natural one. Furthermore, the purified hEGF showed biological activity in the following experiment.

Experiment 2

[Binding ability to EGF receptor]

Following the procedure described below, binding ability of the purified hEGF to human cell surface EGF receptor was confirmed.

Human A431 cells were suspended in Dulbecco's modified Eagle medium (DMEM medium: manufactured by Nissui Co., Ltd.) containing 10% fetal calf serum (manufactured by Gibco Co., Ltd.) in $10^4$ cells/ml. The cell suspension was charged in a 24 well culture plate in 1 ml each per well followed by culturing at 37°C for 24 hours in 5% $CO_2$. After the cells were washed twice with DMEM medium (binding buffer) containing 50 mM N,N-bis (2-hydroxyethyl)-2-aminoethanesulfonic acid (BES, pH 6.5) and 0.1% bovine serum albumin (BSA: manufactured by Sigma Inc.), 0.3 ml of the binding buffer containing 0.2 ng/ml of $^{125}I$-labeled hEGF (manufactured by Amersham Inc.) and 0.1 to 10,000 ng/ml of hEGF were added to the cells.

After allowing to stand at room temperature for 1 hour, the cells were washed twice with the binding buffer and 0.2M sodium hydroxide solution was added in 0.3 ml each per well to lyse the cells. Then, radioactivity of $^{125}I$-labeled hEGF bound to EGF receptor was measured. The results are shown in Table 7.

Table 7

| Binding ability of purified hEGF to human EGF receptor | |
| --- | --- |
| hEGF ng/well/$10^4$ cells | Rate of Binding Inhibition (%) [purified hEGF] |
| 0.1 | 0 |
| 1 | 8 |
| 10 | 38 |
| 100 | 82 |
| 1,000 | 95 |
| 10,000 | 98 |

From the results above, it was confirmed that the purified hEGF had binding ability to EGF receptor of human culture cells.

Experiment 3

[Cell growth accelerating activity[

Following the method described below, the cell growth accelerating activity of the purified hEGF was confirmed.

Mouse Swiss 3T3 cells were suspended in DMEM medium containing 10% fetal calf serum in $10^5$ cells/ml. The cell suspension was charged in a 24 well culture plate in 1 ml each per well and cultured at 37°C in 5% $CO_2$ until the cells reached the intermediate stage of the exponential growth phase. The medium was exchanged with DMEM medium containing 0.2% fetal calf serum followed by further culturing at 37°C in 5% $CO_2$. When almost all cells reached the stationary phase, hEGF was added and culture was continued for further 8 hours. [Methyl-$^3$H] thymidine (247.9 GBq/mmol; manufactured by Amersham Inc.) was added in 0.5 $\mu$Ci each per well. After culturing for 24 hours, radioactivity of [$^3$H] thymidine incorporated into the cells was determined. The results are shown in Table 8.

Table 8

| Cell growth accelerating activity of purified hEGF | | |
|---|---|---|
| Concentration (ng/ml) | Intake of [$^3$H] Thymidine (cpm) | Proportion |
| 0.0 | 2864 | 1.00 |
| 0.3 | 3671 | 1.28 |
| 1.0 | 3753 | 1.31 |
| 3.0 | 4742 | 1.66 |

From the foregoing results, the cell growth accelerating activity of the purified hEGF was confirmed.

Experiment 4

[Inhibition of acid formation in guinea pig gastric wall cell]

Following the method described below, the inhibiting action of the purified hEGF against formation of gastric juice in isolated wall cells was confirmed using $^{14}$C-aminopyrine (AP) accumulation as the index.

After isolated wall cell suspension (1.80 x $10^5$ cells/ml, Hanks solution containing 0.2% BSA (manufactured by Nissui Co., Ltd.)) was kept at 37°C for 20 minutes in 100% oxygen flow, 0.1 $\mu$Ci $^{14}$C-AP (4.37 GBq/mmol; manufactured by Amersham Inc.), purified hEGF and 3 $\mu$M of histamine (manufactured by Nakarai Chemical Co., Ltd.) or 1 mM dibutylyl cyclic-AMP (dbc-AMP, manufactured by Sigma Inc.) as a stimulant were added to the suspension and the mixture was again kept at the same temperature.

Sixty minutes after, 4 ml of ice-cooled Hanks solution was added to the mixture. Then centrifugation at low speed at 4°C was carried out to stop the reaction. The cells were lysed by adding 0.3 ml of 2M sodium hydroxide solution thereto. After neutralizing with 2M hydrochloric acid, its radioactivity was measured by liquid scintillation counter.

Accumulation of $^{14}$C-AP was determined by making as 100% the value obtained by subtracting the control value from the intake value upon the stimulation and the activity of the purified hEGF was expressed by % stimulation. The results are shown in Table 9.

Table 9

| Inhibitory activity of purified hEGF against acid formation | | | |
|---|---|---|---|
| | | | % Stimulation |
| Histamine 3 μM | | | 100 |
| " | + EGF | 1 nM | 105 |
| " | + EGF | 10 nM | 38.1 |
| " | + EGF | 100 nM | 23.1 |
| dbc-AMP 1 mM | | | 100 |
| " | + EGF | 1 nM | 106 |
| " | + EGF | 10 nM | 82.1 |
| " | + EGF | 100 nM | 50.8 |

From the above results, it was confirmed that the purified hEGF also had the activity of inhibiting formation of gastric juice in guinea pig gastric wall cells by stimulation of both histamine and dbc-AMP.

Example 1

[Batch system]

Based on the various findings obtained by the flask culture described above, culture was performed in a large scale, using a jar fermenter of a 10 liter volume. As the jar fermenter, a desk top jar fermenter of a 10 liter volume manufactured by Oriental Yeast Co., Ltd. was used. The jar fermenter was equipped with a control device for regulating pH and dissolved oxygen concentration in medium. HB101 was used as host.

Preculture was performed in LB medium and 2% of the preculture solution was inoculated. As medium, 6 liters of M9 medium (containing 5 g/l Casamino acid, 2 g/l glucose, 10 mg/l vitamin $B_1$ and 100 mg/l ampicillin) was used. Incubation was carried out under conditions of an aeration amount of 1 vvm (volume/volume/minutes; feeding the same amount of air as the culture volume per minute with a air compressor; 6 liters/minute in this case) at 37°C, a dissolved oxygen concentration of 4 ppm (which was controlled by varying the rotation speed of agitation wings between 100 and 1000 rpm), pH of 7.0 (which was controlled by dropwise adding 4M sodium hydroxide solution), and automatic defoaming (defoaming was conducted by detecting a foamed state with a sensor in the upper part of the fermenter and automatically adding a silicone type antifoaming agent dropwise). IAA was added 4.5 hours after and its addition concentration was 20 mg/l.

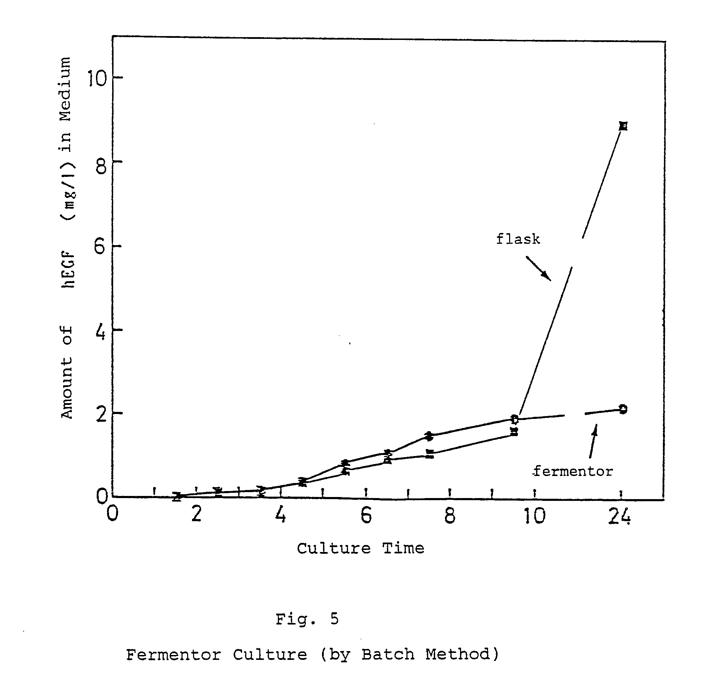

For control, flask culture was carried out in the same medium composition using the same preculture solution under the same conditions as in Reference Example 2. The results are shown in Fig. 5.

In both fermenter culture and flask culture, hEGF production started 4 hours after and continued almost in the same amount up to 9 hours after. However, 24 hours after, 9 mg/l of production was noted in the flask culture whereas in the fermenter culture, 2 mg/l of production which was almost the same as that 9 hours after was merely noted.

Therefore, it is understood that it was impossible to improve the hEGF production amount in conventional batch system. This is believed to be because, in the fermenter culture, the dissolved oxygen amount and pH in the medium were kept constant and the optimum conditions for proliferation of E. coli were always provided so that tryptophan in the medium was consumed and reduced to less than a definite concentration; when trp promoter began to work, nutrient sources (carbon sources, nitrogen sources, etc.) in the medium were exhausted.

Thus, as described below, the fed-batch system in which a fresh medium is continuously supplemented during the course of culture was attempted.

Example 2

[Fed-batch system - part 1]

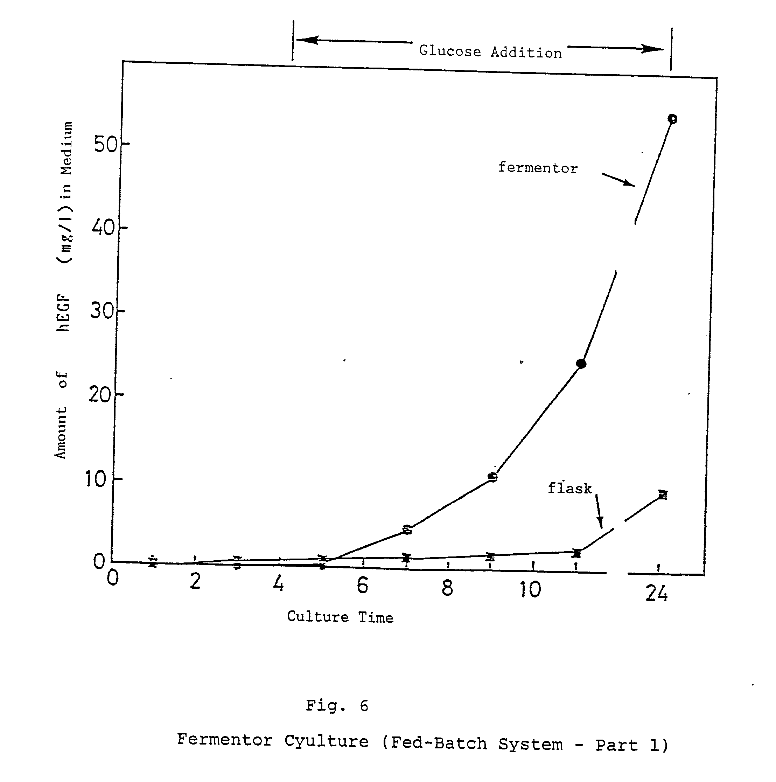

Under the same culture conditions as in Example 1, a supplementing medium (M9 medium containing 20 g/l Casamino acid, 200 g/l glucose, 10 mg/l vitamin $B_1$, 100 mg/l ampicillin and 20 mg/l IAA) was supplemented at a rate of 30 ml/hr for 24 hours using a tube pump, at the same time when IAA was added. For control, flask culture was also carried out. The results are shown in Fig. 6.

In the fermenter culture, hEGF production was increased from 6 hours after and showed 25 mg/l 9 hours after and 55 mg/l 24 hours after, which was about 25 times that of the batch system in Example 1.

However, the maximum was still about 55 mg/l and cell proliferation was about 6 at $OD_{550}$, which results were much farther than the contemplated results in high density culture. Thus, further study was made.

It is known that by previously adding tryptophan in medium, the cell amount increases. Therefore, tryptophan was added to basal medium to inhibit the action of trp promoter so that it was attempted to increase the cell amount. In this case, it was recognized that when tryptophan remained in the medium, it inhibited the action of trp promoter when IAA was added. It was thus desired that tryptophan had been degraded when IAA was added.

On the other hand, glycerol does not cause catabolite repression, unlike glucose. It is also known that when catabolite repression is lost, tryptophanase (tryptophan degradation enzyme) is produced to degrade tryptophan in E. coli.

From the foregoing, glycerol was attempted to use in the supplementing medium, in place of glucose. By doing so, the mechanism that when glucose in basal medium is exhausted, catabolite repression is lost, tryptophanase is produced, tryptophan is used up and hence, trp promoter begins to work is expected.

Furthermore, the addition rate of the supplementing medium is also varied depending upon proliferation of E. coli to attempt elaborated contrivances.

Example 3

[Fed-batch system - part 2]

The medium composition and culture conditions were identical with those of Example 1 except for adding tryptophan to basal medium in 30 mg/l. A supplementing medium (M9 medium containing 5 g/l Casamino acid, 200 g/l glucose, 10 mg/l vitamin $B_1$, 100 mg/l ampicillin and 5 mg/l IA) began to add to the medium from 3 hours after and IAA was added 4 hours after. IAA was added in a concentration of 5 mg/l since an excess amount of IAA would inhibit proliferation of E. coli and hEGF production.

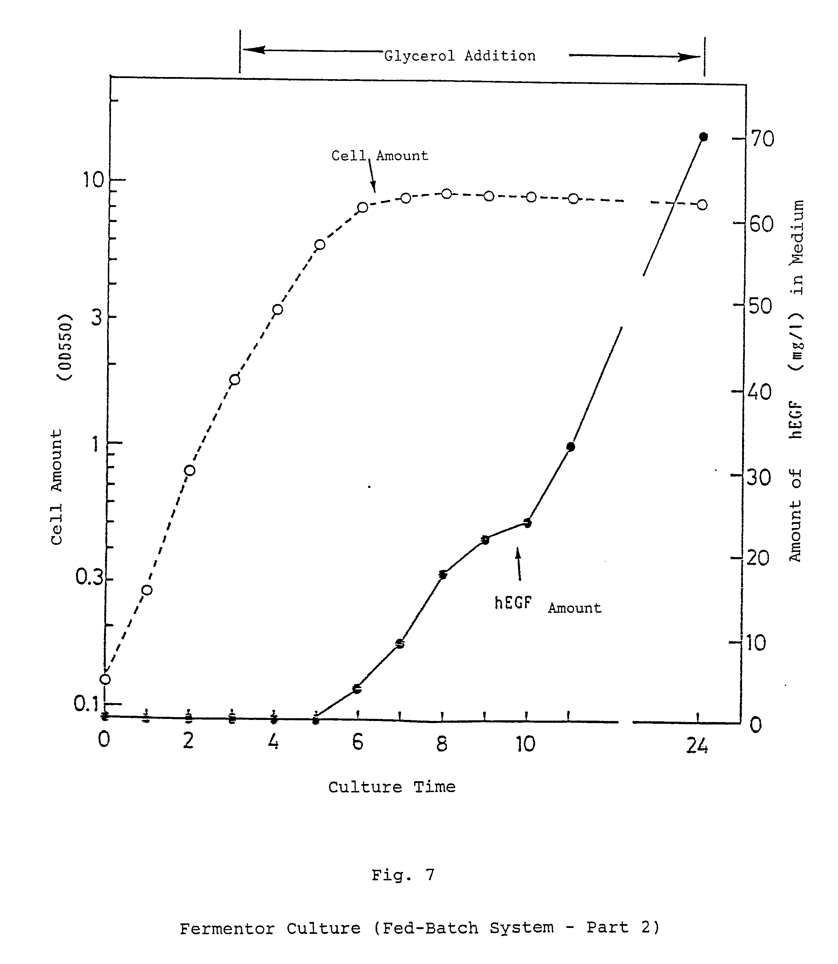

For addition of the medium, PI tube pump (manufactured by Pharmacia Fine Chemicals Inc.) and a personal computer (PC 9801 VX41, manufactured by Nippon Electric Co., Ltd.). A rate of the addition was programmed to make 49 changes in total during the addition. Proliferation of E. coli and hEGF production amount are shown in Fig. 7.

From about 5 hours after when the proliferation rate of E. coli decreased, hEGF production started and reached 70 mg/l 24 hours after. Cell proliferation was also increased to 9 at $OD_{550}$.

From the foregoing results, it is noted that hEGF production amount increases as the cell amount increases.

Thus, in order to perform culture in a higher density, medium composition and timing for addition were studied in detail. Specifically, amino acids necessary for proliferation of host E. coli were added to the medium and the carbon source in the supplementing medium was changed from glucose to glycerol.

As described above, in the presence of glucose, tryptophan is consumed as E. coli proliferates but is not degraded by tryptophanase. It is thus attempted that: glucose and tryptophan are added to the initial supplementing medium to sufficiently proliferate E. coli, while preventing trp promoter from acting. Then, IAA is added and at the same time, the supplementing medium is changed to one containing glycerol so that tryptophan is degraded to strengthen the action of IAA and hence the hEGF production amount is increased.

Example 4

13

[Fed-batch system - part 3]

Culture was carried out as follows, according to the fed-batch system similar to the above.

(1) Medium composition

1) Basal medium

The following were supplemented to M9 medium.
2.5 g/l Casamino acid, 10 g/l glucose, 50 mg/l tryptophan, 0.5 g/l proline, 1.0 g/l leucine, 10 mg/l vitamin $B_1$, 100 mg/l ampicillin and trace metal salts.

2) Supplementing medium 1

The following were supplemented to M9 medium.
40 g/l Casamino acid, 300 g/l glucose, 50 mg/l tryptophan, 5 g/l proline, 5 g/l leucine, 10 mg/l vitamin $B_1$, 100 mg/l ampicillin and trace metal salts.

3) Supplementing medium 2

The following were supplemented to M9 medium.
4.0 g/l Casamino acid, 300 g/1 glycerol, 5 g/1 proline, 5 g/l leucine, 10 mg/l vitamin $B_1$, 100 mg/l ampicillin and trace metal salts.

(2) Culture condition

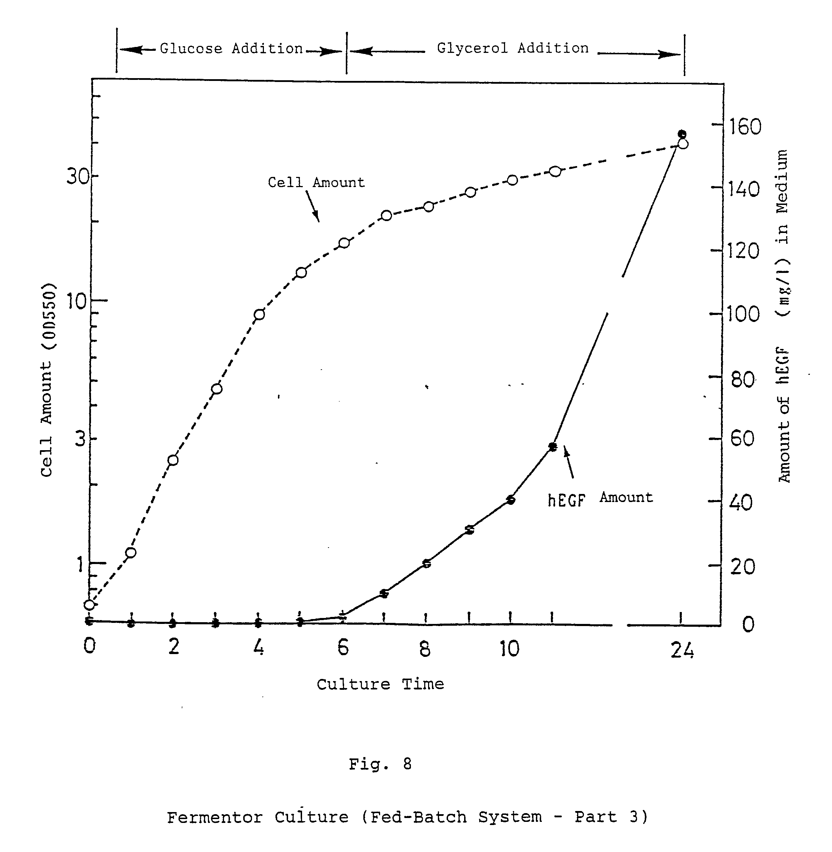

Plasmid (host) was pBT23 (HB101).
Culture volume (basal medium + supplementing medium 1 + supplementing medium 2) was 5 liters + 0.5 liter + 1.0 liter.
Culture temperature was 37° C and pH was 7.0.
Aeration amount was 1.5 vvm (7.5 liters/min).
Number of agitation was 900 rpm.
Defoaming was automated (by dropwise addition of silicone type antifoaming agent).
The timing for addition of medium: supplementing medium 1 was 0.5 to 6 hours (rate of addition was variable) after initiation of the culture and supplementing medium 2 was 6 to 24 hours (rate of addition was constant, 50 ml/hour) after initiation of the culture.
IAA was added 6 hours after initiation of the culture and its concentration was 5 mg/l.
Preculture medium was identical with basal medium.
An inoculation amount was 10%.
A rate of supplying supplementing medium 1 was programmed to make 20 changes in total during the addition. Proliferation of E. coli and hEGF production amount are shown in Fig. 8.
$OD_{550}$ 24 hours after showed 42, indicating that higher density culture was achieved. hEGF was produced in an amount of 160 mg/l.

4. Brief Description of the Drawings

Fig. 1 shows a sequence of the trp promoter in accordance with the present invention, SD sequence and E. coli alkaline phosphatase signal peptide gene.
Fig. 2 shows a sequence of the hEGF gene in accordance with the present invention.
Fig. 3 illustrates the progress of producing the secretion expression plasmid in accordance with the present invention.
Fig. 4 illustrates a sequence of Hind III-BamH I fragment of the secretion expression plasmid pBT23 in accordance with the present invention.

Fig. 5 shows the results of Example 1, wherein the abscissa represents a culture time (hour) and the vertical axis represents an amount (mg/l) of hEGF in medium: ● denotes culture in a fermenter and ■ denotes flask culture.

Fig. 6 shows the results of Example 2, wherein the abscissa represents a culture time (hour) and the vertical axis represents an amount (mg/l) of hEGF in medium: ● denotes culture in a fermenter and ■ denotes flask culture.

Fig. 7 shows the results of Example 3, wherein the abscissa represents a culture time (hour) and the vertical axis represents an amount (mg/l) of hEGF and cell amount (expressed by $OD_{550}$) in medium: o denotes cell amount and ● denotes hEGF production.

Fig. 8 shows the results of Example 4, wherein the abscissa represents a culture time (hour) and the vertical axis represents an amount (mg/l) of hEGF and cell amount (expressed by $OD_{550}$) in medium: o denotes cell amount and ● denotes hEGF production.

## Claims

(1) In a genetic engineering technology which comprises performing a series of procedures (1) through (4): (1) integrating a gene coding for an objective substance in a vector, (2) transforming host E. coli using this vector, (3) culturing the thus obtained transformant, and (4) recovering and collecting the objective substance from the culture solution:
a process for producing hEGF characterized by (a) and (b) below:

(a) said objective substance is hEGF;

(b) in said procedure (1) above, a gene encoding a promoter for tryptophan (trp) operon and a gene encoding a signal peptide for gene of E. coli alkaline phosphatase are integrated into a hEGF gene in the upstream region and continuous two termination codons and a transcription terminator are integrated in the downstream region.

(2) A process for producing hEGF according to claim (1), wherein 3-indoleacrylic acid (IAA) is added during the course of culture, whereby the time period and amount of hEGF produced by host E. coli are controlled.

(3) A process for producing hEGF according to claim (2), wherein said culture is high density culture efficiently utilizing fed-batch system in which a rate of addition is contrived.

(4) A process for producing hEGF according to claim (3), wherein glucose or glycerol or glucose and glycerol in combination are used as carbon sources for a supplementing medium in the fed-batch system.

(5) A process for producing hEGF according to claim (3), wherein glucose is used and then glycerol is used, as carbon sources for a supplementing medium in the fed-batch system.

```
                                                    ─── trp  Promotor ───────────
HindIII
AGCTTTGGCAAATATTCTGAAATGAGCTGTTGACAATTAATCATCGAACTAGTTAACTAG
      AACCGTTTATAAGACTTTACTCGACAACTGTTAATTAGTAGCTTGATCAATTGATC


                          SD  Sequence      ┌── E. coli alkaline phosphat-
TACGCAAGTTCACGTAAAAAGGGTATCGACAATGAAACAAAGCACTATTGCACTGGCACT
ATGCGTTCAAGTGCATTTTCCCATAGCTGTTACTTTGTTTCGTGATAACGTGACCGTGA


ase signal peptide gene
                                         EcoRI
CTTACCGTTACTGTTTACCCCTGTGACAAAAGCG
GAATGGCAATGACAAATGGGGACACTGTTTCGCTTAA
```

Fig. 1    Sequence of trp promotor, SD sequence
and E. coli alkaline phosphatase signal
peptide gene

EcoRI
AATTCCGACTCTGAATGTCCGCTGTCTCATGATGGTTACTGTCTGCACGATGGTGTTTGT
GGCTGAGACTTACAGGCGACAGAGTACTACCAATGACAGACGTGCTACCACAAACA

————————————————————————————————— hEGF gene ——————
SphI
ATGTACATCGAAGCACTGGATAAATACGCATGCAATTGTGTAGTTGGTTACATCGGTGAA
TACATGTAGCTTCGTGACCTATTTATGCGTACGTTAACACATCAACCAATGTAGCCACTT

SacI
CGTTGCCAATACCGTGATCTGAAATGGTGGGAACTGCCGCTGATAGAGCT
GCAACGGTTATGGCACTAGACTTTACCACCCTTGACGCGACTATC

Fig. 2    Sequence of hEGF gene

Fig. 3  Production of secretion expression plasmid

HindIII ⌐――――――――――――――――――――――― trp. Promotor ――
AGCTTTGGCAAATATTCTGAAATGAGCTGTTGACAATTAATCATCGAACTAGTTAACTAG
AACCGTTTATAAGACTTTACTCGACAACTGTTAATTAGTAGCTTGATCAATTGATC

――――――――――――――――――――――⌐⌐― E. coli alkaline phosphat-
SD sequence
TACGCAAGTTCACGTAAAAAGGGTATCGACAATGAAACAAAGCACTATTGCACTGGCACT
ATGCGTTCAAGTGCATTTTTCCCATAGCTGTTACTTTGTTTCGTGATAACGTGACCGTGA

ase signal peptide gene ――――――――――――
EcoRI
CTTACCGTTACTGTTTACCCCTGTGACAAAAGCGAATTCCGACTCTGAATGTCCGCTGTC
GAATGGCAATGACAAATGGGGACACTGTTTTCGCTTAAGGCTGAGACTTACAGGCGACAG

――――――――――――――― hEGF gene ――
TCATGATGGTTACTGTCTGCACGATGGTGTTTGTATGTACATCGAAGCACTGGATAAATA
AGTACTACCAATGACAGACGTGCTACCACAAACATACATGTAGCTTCGTGACCTATTTAT

SphI
CGCATGCAATTGTGTAGTTGGTTACATCGGTGAACGTTGCCAATACCGTGATCTGAAATG
GCGTACGTTAACACATCAACCAATGTAGCCACTTGCAACGGTTATGGCACTAGACTTTAC

――――――――――⌐ trpA. terminator ――
SacI
GTGGGAACTGCGCTGATAGAGCTCAGCCCGCCTAATGAGCGGGCTTTTTTTCTG
CACCCTTGACGCGACTATCTCGAGTCGGGCGGATTACTCGCCCGAAAAAAAGACCTAG
BamHI

Fig. 4   Sequence of Hind III-Bam IL fragment
of Secretin Expression Plasmid pBT23

Fig. 5

Fermentor Culture (by Batch Method)

Fig. 6

Fermentor Cyulture (Fed-Batch System - Part 1)

Fig. 7

Fermentor Culture (Fed-Batch System - Part 2)

Fig. 8

Fermentor Culture (Fed-Batch System - Part 3)